# EUROPEAN PATENT APPLICATION

(11) **EP 1 036 553 A2**
(43) Date of publication of application: **20.09.2000**
(21) Application number: 00105666.2
(22) Date of filing: 17.03.2000
(51) Int. Cl.: A61K 7/02

(54) **Solid water-in-oil type emulsion cosmetic composition**

(30) Priority: 18.03.1999 JP 7443199
(71) Applicant: SHISEIDO COMPANY LIMITED, Chuo-ku, Tokyo 104-8010 (JP)
(72) Inventor: Daisuke, Aso, c/o Shiseido Research Center, Yokohama-shi, Kanagawa 223-8553 (JP); Akihito, Yokotsuka, c/o Shiseido Research Center, Yokohama-shi, Kanagawa 223-8553 (JP)
(74) Representative: Henkel, Feiler, Hänzel

(57) **Abstract**

A solid water-in-oil type emulsion cosmetic composition having a fresh, superior skin feeling, and effectively correcting roughness of the skin while exhibiting long-lasting power, which contains a hydrophobically treated powder, spherical silica, an oil, and water, wherein a ratio by weight of the oil to the water (i.e. oil/water) is 0.5 to 10.

## Description

### BACKGROUND OF THE INVENTION

### 1. Field of the Invention

The present invention relates to a solid water-in-oil type emulsion cosmetic composition. More specifically it relates to a solid water-in-oil type emulsion cosmetic composition having a superior skin feeling having a freshness, when applied to, for example, the face as a cosmetic, having a superior long-lasting power against sweat and skin oil, and capable of hiding roughness of the skin surface (pores etc.), particularly it relates to a solid water-in-oil type emulsion cosmetic composition suitable as a make-up cosmetic composition.

### 2. Description of the Related Art

In conventional cosmetic compositions, spherical powder is often formulated therein for the purpose of eliminating or hiding shine after application or roughness of the skin surface (specifically, pores etc.). However, in the case of emulsion cosmetic compositions of a water-in-oil type, there is the problem that a large amount of spherical powder for imparting the above effect while trying to maintain the "fresh" skin feel leads to an increase in the total amount of powder, and therefore, a higher viscosity at high temperatures, decline in fluidity, and, therefore, difficulty in filling into containers. Further, when increasing the amount of the spherical powder blended without changing the total amount of powder, the covering power or a feeling that fit on skin tends to be reduced.

### SUMMARY OF THE INVENTION

The present invention was made in consideration of the above-mentioned conventional situation in the art and the object thereof is to provide a solid water-in-oil type emulsion cosmetic composition having a sufficiently satisfactory skin feel, in particular freshness, and simultaneously superior in correction of skin roughness, particularly a solid water-in-oil type emulsion cosmetic composition for a make-up cosmetic composition.

The present inventors engaged in intensive studies to solve the above-mentioned problems and, as a result, found that, by formulating a hydrophobically treated powder, spherical silica, an oil, and water in the specific ratio, it is possible to obtain a solid water-in-oil type emulsion cosmetic composition superior in freshness and other skin feeling and superior in the effect of improvement of the skin, whereby the present invention has been completed.

In accordance with the present invention, there is provided a solid water-in-oil emulsion cosmetic composition comprising: a hydrophobically treated powder, spherical silica, an oil, and water, in which the ratio by weight of the oil to the water (i.e., oil/water) is 0.5 to 10.

### DESCRIPTION OF THE PREFERRED EMBODIMENTS

The present invention will be explained in detail below.

The "hydrophobically treated powder" used in the present invention means those composed of a powder, which can generally be formulated into a cosmetic composition, covered with various organic compounds and may be obtained by the physical adsorption, chemical bonding, etc. to the powder. Further, the hydrophobically treated powder in the present invention means those which, when 0.5 g of the treated powder is placed in 100 ml of deionized water, and is allowed to stand at 50°C for 1 hour, the majority of the powder is found to be floating on the water surface by a visual evaluation.

The hydrophobic treatment agent for such a hydrophobically treated powder includes, a hydrophobic compound such as for example, a metal soap, oil and fat, wax, silicone compound, fluorine compound, hydrocarbon compound, fatty acid ester, lyophilic surfactant may be mentioned. In the present invention, these compounds may be used alone or in any combinations thereof. Further, as the powder to be hydrophobically treated, those explained later are suitably selected for use.

The hydrophobic treatment agents particularly preferred in the present invention are silicone compounds, metal soaps, and fluorine compounds. More preferred are silicone compounds. Especially, the use of the treated powder having a high or increased coverage density of a silicone compound with an Si-H group (preferably alkylated) on the surface thereof are preferable.

The formulating amount of the hydrophobically treated powder is 5.0 to 60.0 % by weight, preferably 10.0 to 50.0 % by weight, more preferably 15.0 to 45.0 % by weight. If the amount of the hydrophobically treated powder is less than 5.0 % by weight, the covering power, fit, etc. tend to decrease, while if more than 60.0 % by weight, the smooth spreadability at the time of application and freshness tend to be decreased and the skin feel becomes worse.

The spherical silica usable in the present invention includes any silica having a so-called spherical shape preferably having a diameter of 0.1 - 30 µm, more preferably 1 - 10 µm. The surface of the spherical silica may be porous or nonporous, although the use of porous spherical silica is preferable, because the nonporous silica takes an aqueous component in such a manner that the aqueous component forms a film around the spherical powder. Contrary to this, the porous spherical silica powder adsorbs the aqueous component. The formulating amount of the spherical silica is 2.0 to 15.0 % by weight, preferably 4.0 to 15.0 % by weight, more preferably 5.0 to 10.0 % by weight, in view of, for example, the feeling of use at the time of application. If the amount of the spherical silica is less than 2.0 % by weight, the fillability in containers becomes worse and the smoothness after application also becomes poor. Further, if more than 15.0 % by weight, the decrease in a feeling that fit on skin at the time of application, a slipperiness or sliminess are observed - which are not desirable in views of skin feel.

Examples of spherical powders, other than spherical silica, capable of formulating in the present invention are polyalkyl methacrylate powder, nylon powder, polystyrene powder, polyurethane powder, cellulose powder, cross-linkable silicone powder. Among these powders, cross-linkable silicone powder and polyalkyl methacrylate powder are particularly preferable.

The proportion of the spherical silica formulated in the total spherical powder is 30 to 100 % by weight, particularly 50 to 100 % by weight. If the proportion is less than 30 % by weight, the problem during the manufacture occurs since the filling the composition into a container becomes difficult due to the increase in the viscosity at an elevated temperature and also the problem from the functional viewpoint occurs since the correction of skin roughness with the cosmetic composition is not sufficient. In addition, when the spherical silica is formulated into the cosmetic composition, the spherical silica is preferably formulated after adsorbing an aqueous component (e.g., water, hemectant etc.) in view of the applicability and the stability. Such an adsorption can be effected simply by contacting the spherical silica with the aqueous component.

The oil formulated into the solid water-in-oil type emulsion cosmetic composition of the present invention includes any oils usually used in cosmetic compositions. For example, as liquid oils, avocado oil, tsubaki oil, macademia nut oil, mink oil, olive oil, rapeseed oil, jojoba oil, triglycerin, glycerin trioctanate, etc.; as solid oils and fats, coconut oil, hydrogenated coconut oil, palm oil, beef tallow, sheep fat, Japan wax, hydrogenated castor oil, etc.; as waxes, beeswax, candelilla wax, carnauba wax, insect wax, spermaceti, lanolin, reduced lanolin, etc.; as hydrocarbons, liquid paraffin, squalane, paraffin, ceresine, vaseline, squalene, microcrystalline wax, etc.; as higher fatty acids, lauric acid, myristic acid, palmitic acid, stearic acid, behenic acid, oleic acid, 12-hydroxystearic acid, isostearic acid, linolic acid, linoleic acid, etc.; as higher alcohols, lauryl alcohol, cetyl alcohol, stearyl alcohol, behenyl alcohol, oleyl alcohol, monostearyl glycerol ether, monopalmityl glycerol ether, cholesterol, phytosterol, isostearyl alcohol, etc.; as ester oils, isopropyl myristate, cetyl octanate, octyldodecyl myristate, butyl stearate, decyl oleate, ethyleneglycol dioctanate, diisostearyl malate, trimethylolpropane trioctanate, trimethylolpropane triisostearate, pentanerythritol tetraoctanate, glycerin trioctanate, glycerin triisostearate, ethyl acetate, butyl acetate, amyl acetate, etc.; as silicones, dimethyl polysiloxane, methylphenyl polysiloxane, methylhydrogen polysiloxane, decamethyl cyclopentasiloxane, octamethyl cyclotetrasiloxane, silicone resins forming three dimensional network structures, silicone rubber, etc. may be mentioned, but the present invention is not limited to the above-mentioned oils. Further, these oils may be used alone or in any combination thereof in the solid water-in-oil type emulsion cosmetic composition of the present invention.

The ratio of the oil to the water formulated in the solid water-in-oil type emulsion cosmetic composition is a ratio of the oil to water (i.e., oil/water) of 0.5 to 10 (ratio by weight), preferably 1 to 5. If this ratio is less than 0.5, the viscosity becomes higher at a high temperature, the fluidity is decreased, and filling in a container tends to become more difficult. If the ratio is more than 10, the skin feeling, that is, the freshness at the time of application, becomes inferior.

Further, the sum of the proportions of the oil and water formulated in the solid water-in-oil type emulsion cosmetic composition is preferably 40.0 to 90.0 % by weight. If this amount of sum is less than 40.0 % by weight, the viscosity becomes higher at a high temperature, the fluidity is decreased, and filling in a container tends to become more difficult. If the amount is more than 90.0 % by weight, it is not possible to sufficiently impart the effect of smoothness after application and correcting roughness of the skin surface.

The solid water-in-oil type emulsion cosmetic composition of the present invention may have suitably formulated it, in addition to the above components, optionally, a powder other than the hydrophobically treated powder, surfactant, lower alcohol, polyhydric alcohol, humectant, preservative, polymer other than a coating agent, antioxidant, UV blocker, fragrance, various medicines, etc. in a range of quality and quantity, which do not impair the above effects of the present invention.

As the powder capable of formulating into the cosmetic compositions of the present invention, powders usually used in cosmetic compositions may be mentioned. Examples of such powder are inorganic powders such as talc, kaolin, mica, sericite, dolomite, biotite, phlogopite, synthetic mica, vermiculite, magnesium carbonate, calcium carbonate, aluminum silicate, barium silicate, calcium silicate, magnesium silicate, metal salts of tungstic acid, magnesium, silica, zeolite, barium sulfate, sintered calcium sulfate, sintered gypsum, calcium phosphate, fluorapatite, hydroxyapatite, ceramic powder, and metal soaps (e.g., zinc myristate, calcium palmitate, and aluminum stearate); organic powders such as polyamide resin powder, polyethylene powder, methyl polymethacrylate powder, polystyrene powder, styrene and acrylic acid copolymer resin powder, benzoguanamin resin powder, polytetrafluoroethylene powder, cellulose powder; inorganic white pigments such as titanium dioxide and zinc oxide; inorganic red pigments such as iron oxide (e.g. bengara) and iron titanate; inorganic brown pigments such as γ-iron oxide; inorganic yellow pigments such as yellow iron oxide and yellow earth; inorganic black pigments such as black iron oxide, carbon black, and lower titanium oxide; inorganic violet pigments such as mango violet and cobalt violet; inorganic green pigments such as chromium oxide, chromium hydroxide, and cobalt titanate; inorganic blue pigments such as prussian blue and ultramarine; pearl pigments such as titanium oxide coated mica, titanium oxide coated bismuth oxichloride, titanium oxide coated talc, colored titanium oxide coated mica, bismuth oxichloride, and fish scales; metal powder pigments such as aluminum powder and copper powder; lakes such as Lithol rubine BCA (Red No. 202), Lithol red (Red No. 205), Deep maroon (Red No. 220), Permatone Red (Red No. 228), Permanent red F5R (Red No. 405), Permanent orange (Orange No. 203), Benzidine Orange (Orange No. 204), Benzidine yellow G (Yellow No. 205), Hanza Yellow (Yellow No. 401), and Yellow AB (Blue No. 404); zirconium, barium, or aluminum lakes and other lakes such as Erythrosine (Red No. 3), Phloxine B (Red No. 104), Fast acid magenta (Red No. 227), Violamine R (Red No. 401), Orange II (Orange No. 205), Tartrazine (Yellow No. 4), (Yellow No. 202), Fast green FCF (Green No. 3), and Brilliant blue FCF (Blue No. 1); and natural colors such as chlorophyl and β-carotin may be mentioned.

In the solid water-in-oil type emulsion cosmetic composition of the present invention, it is possible to use a surfactant usually capable of formulating in a cosmetic compositions, without regard as to its ionic character. Specifically, as anionic surfactants, for example, soap raw materials, fatty acid soaps such as sodium laurate, higher alkyl sulfate ester salts such as sodium laurosulfate, alkyl ether sulfate ester salts such as polyoxyethylene (i.e., "POE") laurosulfate triethanol amine, N-acylsarcosine acids such as sodium lauroyl sarcosinate, higher fatty acid amide sulfonates such as sodium N-cocoyl-N-methyl taurate, phosphate ester salts such as POE stearyl ether phosphate, sulfosuccinates such as sodium di-2-ethylhexylsulfosuccinate, alkylbenzensulfonates such as sodium dodecylbenzensulfonate, N-acyl glutamates such as disodium N-stearoyl glutamate, higher fatty acid ester sulfate ester salts such as sodium hydrogenated glyceryl cocoate sulfate, sulfated oils such as Turkey red oil, POE alkyl ether carboxylic acid, POE alkylaryl ether carboxylate, α-olefinsulfates, higher fatty acid ester sulfonates, secondary alcohol sulfate ester salts, higher fatty acid alkylolamide sulfate ester salts, sodium lauroyl monoethanolamide succinate, N-palmitoyl asparaginate ditriethanol amine, sodium caseine, etc. may be mentioned.

As cationic surfactants, for example, alkyl trimethyl ammonium salts such as stearyl trimethyl ammonium chloride, dialkyldimethyl ammonium salts such as distearyldimethyl ammonium chloride, alkyl pyridinium salts such as cetylpyridinium chloride, alkyl quaternary ammonium salts, alkyl dimethylbenzyl ammonium salts, alkyl isoquinolinium salts, dialkyl morphonium salts, POE alkyl amines, alkyl amine salts, polyamine fatty acid derivatives, amyl alcohol fatty acid derivatives, benzalkonium chloride, benzethonium chloride, etc. may be mentioned.

As bipolar surfactants, for example, imidazoline base bipolar surfactants such as sodium 2-undecyl-N,N,N-(hydroxyethylcarboxymethyl)-2-imidazoline, betaine base surfactants such as lauryldimethyl-aminoacetate betaine, etc. may be mentioned.

As lypophilic nonionic surfactants, for example, sorbitan fatty acid esters such as sorbitan monoisostearate and sorbitan sesquioleate, glyceryl polyglyceryl fatty acids such as glyceryl monostearate, propylene glycol fatty acid esters such as propylene glycol monostearate, hydrogenated castor oil derivatives, glyceryl alkyl ethers, dimeticone copolyol, alkyl dimeticone copolyol etc. may be mentioned

As hydrophilic nonionic surfactants, for example, POE sorbitan fatty acid esters such as POE sorbitan monostearate, POE sorbite fatty acid esters such as POE-sorbite monooleate, POE glyceryl fatty acid esters such as POE glyceryl monoisostearate, POE alkyl ethers such as POE stearyl ethers and POE cholestanol ethers, POE alkyl phenyl ethers such as POE nonyl phenyl ethers, pluaronics such as Pluronic, POE·POP alkyl ethers such as POE·polyoxypropylene (i.e., "POP") cetyl ethers, tetra POE·tetra POP ethylene diamine condensation products such as Tetronic, POE castor oil and hydrogenated castor oil derivatives such as POE castor oil and POE hydrogenated castor oil, POE beeswax·lanolin derivatives, alkanolamides, POE propylene glycol fatty acid esters, POE alkylamines, POE fatty acid amides, sucrose fatty acid esters, POE nonylphenyl formaldehyde condensation products, alkylethoxydimethylamineoxide, trioleylphosphoric acid etc. may be mentioned, but the invention is not limited to the above surfactants.

Further, in the solid water-in-oil emulsion cosmetic of the present invention, these surfactants may be used alone or in any combinations thereof.

The use of dimeticone copolyol, alkyl dimeticone copolyol, sorbitan fatty acid esters, POE sorbitan fatty acid esters, POE hydrogenated castor oil, glyceryl fatty acid esters, POE glyceryl fatty acid esters, etc, is especially preferable as the surfactant due to the easy filling ability to a container (i.e., preferable elevated temperature characteristics), the good feeling during the application (i.e., no stickiness), the good emulsion stability, etc.

The form which the solid water-in-oil type emulsion cosmetic composition of the present invention takes is not particularly limited. The cosmetic composition may take the form of, for example, a cosmetic base, foundation, face powder, blusher, lipstick, mascara, eyeshadow, or eyeliner.

### EXAMPLES

Preferable Examples of the present invention will be explained below, but the present invention is not limited thereto. Further, the amounts blended are shown by % by weight, unless otherwise indicated.

### Examples 1 to 2 and Comparative Examples 1 to 4

Solid water-in-oil emulsion type foundations were prepared by the method explained below according to the formulations shown in the following Table 1 and Table 2 and were evaluated as to various performance as cosmetic compositions. The method of evaluation was as explained below. The results are shown in Table 3.

### 1. Method of Preparation

The components (1) to (6) shown in Table 1 and Table 2 were heated and mixed homogeneously at 90°C. The mixed and pulverized components (7) to (12) were then added thereto to obtain homogeneously dispersed mixture. The homogeneously melted and dispersed components (13) to (25) were then added to obtain a homogeneous emulsion. Next, the emulsion was filled in a pan, followed by cooling.

### 2. Method of Evaluation

The solid water-in-oil type emulsion foundation prepared was subjectively evaluated by an expert panel of 10 members based on the following standard for evaluation as to whether it had a light spreadability on the skin, freshness at time of application, smoothness after application, effect in correcting skin roughness, long-lasting power after three hours after application, and ease of fillability in a container.

### (1) Standard for Evaluation of Light Spreadability on the Skin, Freshness at Time of Application, Smoothness After Application, Effect in Correcting Skin Roughness, and Long-Lasting Power after 3 Hours from Application

++ (Excellent):At least nine members of panel felt effects.
+ (Good): Seven to eight members of panel felt effects.
± (Fair): Four to six members of panel felt effects.
- (Poor): Three or less members of panel felt effects.

### (2) Standard for Evaluation of Fillability in Container

++ (Excellent):No problem in fluidity when pouring and smooth surface after filling.
+ (Good): Shaking or other procedure required when filling to obtain smooth surface.
± (Fair): Cannot fill smoothly even with shaking or other procedure.
- (Poor): No fluidity and pan cannot be filled.

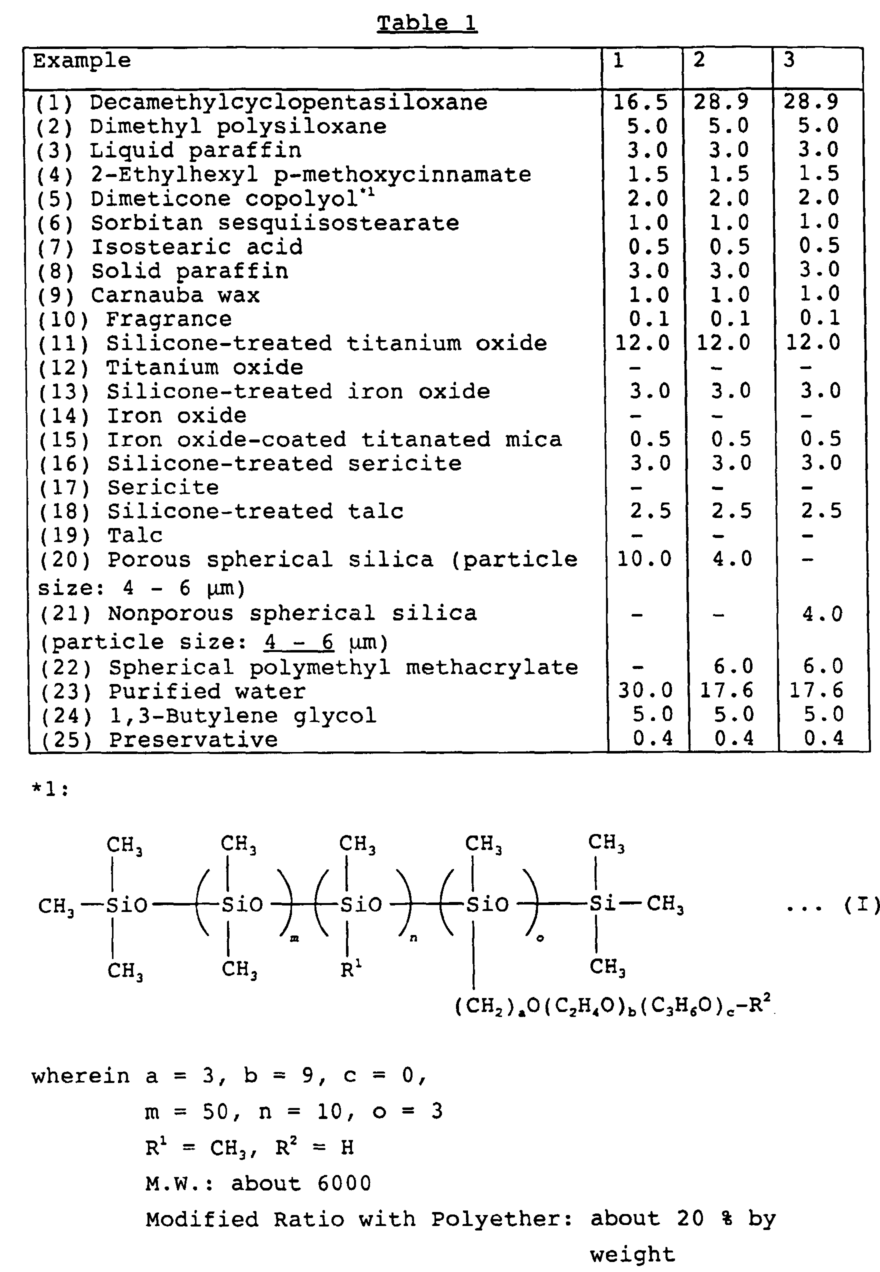

**Table 2**

| Comparative Example | 1 | 2 | 3 | 4 | 5 |
|---|---|---|---|---|---|
| (1) Decamethylcyclopentasiloxane | 16.5 | 16.5 | 55.5 | 2.5 | 16.5 |
| (2) Dimethyl polysiloxane | 5.0 | 5.0 | 5.0 | 5.0 | 5.0 |
| (3) Liquid paraffin | 3.0 | 3.0 | 3.0 | 3.0 | 3.0 |
| (4) 2-Ethylhexyl p-methoxycinnamate | 1.5 | 1.5 | 1.5 | 1.5 | 1.5 |
| (5) Dimeticone copolyol*¹ | 2.0 | 2.0 | 2.0 | 2.0 | 2.0 |
| (6) Sorbitan sesquiisostearate | 1.0 | 1.0 | 1.0 | 1.0 | 1.0 |
| (7) Isostearic acid | 0.5 | 0.5 | 0.5 | 0.5 | 0.5 |
| (8) Solid paraffin | 3.0 | 3.0 | 3.0 | 3.0 | 3.0 |
| (9) Carnauba wax | 1.0 | 1.0 | 1.0 | 1.0 | 1.0 |
| (10) Fragrance | 0.1 | 0.1 | 0.1 | 0.1 | 0.1 |
| (11) Silicone-treated titanium oxide | - | 12.0 | 12.0 | 12.0 | 12.0 |
| (12) Titanium oxide | 12.0 | - | - | - | - |
| (13) Silicone-treated iron oxide | - | 3.0 | 3.0 | 3.0 | 3.0 |
| (14) Iron oxide | 3.0 | - | - | - | - |
| (15) Iron oxide-coated titanated mica | 0.5 | 0.5 | 0.5 | 0.5 | 0.5 |
| (16) Silicone-treated sericite | - | 3.0 | - | 3.0 | 3.0 |
| (17) Sericite | 3.0 | - | - | - | - |
| (18) Silicone-treated talc | - | 2.5 | - | 2.5 | 2.5 |
| (19) Talc | 2.5 | - | - | - | - |
| (20) Porous spherical silica (partical size: 4 - 6 µm) | 10.0 | - | 5.0 | 10.0 | - |
| (21) Non spherical silica (particle size: 4 - 6 µm) | - | - | - | - | 10.0 |
| (22) Spherical polymethyl methacrylate | - | 10.0 | - | - | - |
| (23) Purified water | 30.0 | 30.0 | 3.5 | 45.2 | 30.0 |
| (24) 1,3-Butylene glycol | 5.0 | 5.0 | 3.0 | 5.0 | 5.0 |
| (25) Preservative | 0.4 | 0.4 | 0.4 | 0.4 | 0.4 |

| | | | | | |
|---|---|---|---|---|---|
| ∗1: See Formula (I) in Table 1, wherein a = 3, b = 9, c = 0, m = 50, n = 10, o = 3, R¹ = CH₃, R² = H M.W.: about 6000 Modified Ratio with Polyether: about 20 % by weight | | | | | |

**Table 3**

| | Example | | | Comparative Examples | | | | |
|---|---|---|---|---|---|---|---|---|
| | 1 | 2 | 3 | 1 | 2 | 3 | 4 | 5 |
| Light spreadability | ++ | ++ | ++ | ++ | ++ | ++ | ++ | ± |
| Freshness | ++ | ++ | + | ++ | + | - | ++ | + |
| Smoothness after application | ++ | + | + | ++ | ± | + | + | - |
| Effect in correcting skin roughness | ++ | ++ | ++ | ++ | ++ | + | ++ | - |
| Long-lasting power | ++ | ++ | + | - | ++ | ++ | ++ | ++ |
| Fillability in container | ++ | ++ | + | + | ± | ++ | - | + |

As is clear from the results shown in Table 3, Comparative Example 1 did not achieve cosmetic staying power compared with Examples 1 and 2 since it did not contain a hydrophobically treated powder. Further, Comparative Example 2 resulted in the decrease in the smoothness after application and fillability in a container since spherical silica was not contained. Further, Comparative Examples 3 and 4 did not have suitable ratios of the oil and water, and therefore, Comparative Example 3 was inferior in freshness and Comparative Example 4 was inferior in fillability in a container.

As opposed to the above, Examples 1 and 2 contained suitable amounts of the hydrophobically treated powder, spherical silica, oil, and water, and therefore, all superior in terms of light slip on the skin, freshness at the time of application, smoothness after application, effect of correcting skin roughness, cosmetic staying power after three hours after application, and fillability in a container were obtained. Further, although Example 2 contained spherical PMMA powder in addition to the spherical silica, but the amount blended was suitable, the effect of the present invention was not impaired.

### Example 4

The following components (1) to (17) were used to prepare a cosmetic base as a solid water-in-oil type emulsion cosmetic composition according to the present invention:

| | |
|---|---|
| (1) Dimethyl polysiloxane | 29.0 wt% |
| (2) Methylphenyl polysiloxane | 10.0 |
| (3) Liquid paraffin | 5.0 |
| (4) Glyceryl trioctanate | 5.0 |
| (5) Microcrystallline wax | 2.0 |
| (6) Carnauba wax | 2.0 |
| (7) Sorbitan sesquiisostearate | 3.0 |
| (8) Polyoxyethylene hydrogenated castor oil | 0.5 |
| (9) Silicone-treated titanium oxide | 3.0 |
| (10) Silicone-treated iron oxide | 1.5 |
| (11) Silicone-treated barium sulfate | 4.5 |
| (12) Boronitride | 1.0 |
| (13) Spherical silica | 7.0 |
| (14) Spherical silicone resin powder | 2.0 |
| (15) Purified water | 20.0 |
| (16) Dipropylene glycol | 8.0 |
| (17) Preservative | 0.5 |

### Method of Preparation

The components (1) to (8) were heated to 90°C to melt, then the mixed and pulverized components (9) to (14) were added and stirred to disperse them homogeneously. The homogeneously melted components (15) to (17) were added, then the overall mixture was homogeneously emulsified, then filled in a container and cooled to obtain the solid water-in-oil type emulsion cosmetic base.

### Example 5

The following components (1) to (16) were used to prepare a stick cosmetic composition as a solid water-in-oil type emulsion cosmetic composition according to the present invention:

| | |
|---|---|
| (1) Decamethylcyclopentasiloxane | 16.0 wt% |
| (2) Dodecamethylcyclohexasiloxane | 8.0 |
| (3) Alkyl dimeticone copolyol*¹ | 3.0 |
| (4) Solid paraffin | 5.0 |
| (5) Candelilla wax | 1.0 |
| (6) Carnauba wax | 1.0 |
| (7) Silicone-treated titanium oxide | 15.0 |
| (8) Silicone-treated iron oxide | 3.0 |
| (9) Spherical silica | 10.0 |
| (10) Lauroyllysine powder | 2.0 |
| (11) Metal soap-treated talc | 15.0 |
| (12) Purified water | 10.5 |
| (13) Sodium hydroxymethoxybenzophenone sulfonate | 3.0 |
| (14) Glycerol | 2.0 |
| (15) Dipropylene glycol | 5.0 |
| (16) Preservative | 0.5 |

| | |
|---|---|
| ∗1: See Formula (I) in Table 1, wherein a = 3, b = 50, c = 60, m = 100, n = 50, o = 15, R¹ = C₁₆H₃₃, R² = H M.W.: about 15000 | |

### Method of Preparation

The components (1) to (6) were heated to 90°C to melt, then the mixed and pulverized components (7) to (11) were added and stirred to disperse them homogeneously. The homogeneously melted components (12) to (16) were added, then the overall mixture was homogeneously emulsified, then filled in a container and cooled to obtain the solid water-in-oil type emulsion stick cosmetic composition.

### Example 6

The following components (1) to (17) were used to prepare a solid water-in-oil type emulsion foundation according to the present invention:

| | |
|---|---|
| (1) Dimethyl polysiloxane | 6.0 wt% |
| (2) Decamethylcyclopentasiloxane | 22.0 |
| (3) Dodecamethylcyclohexasiloxane | 12.0 |
| (4) Methylphenyl polysiloxane | 5.0 |
| (5) Microcrystallline wax | 2.0 |
| (6) Carnauba wax | 2.0 |
| (7) Polyether-modified dimethyl polysiloxane*¹ | 3.0 |
| (8) Polyoxyethylene hydrogenated castor oil | 0.5 |
| (9) Silicone-treated titanium oxide | 5.0 |
| (10) Silicone-treated iron oxide | 2.5 |
| (11) Silicone-treated barium sulfate | 1.5 |
| (12) Boronitride | 1.0 |
| (13) Spherical silicone resin powder | 2.0 |
| (14) Spherical silica | 7.0 |
| (15) Purified water | 20.0 |
| (16) Dipropylene glycol | 8.0 |
| (17) Preservative | 0.5 |

| | |
|---|---|
| ∗1: See Formula (I) in Table 1, wherein a = 3, b = 9, c = 0, m = 50, n = 10, o = 3, R¹ = CH₃, R² = H M.W.: about 6000 Modified Ratio with Polyether: about 20 % by weight | |

### Method of Preparation

The components (1) to (8) were heated to 90°C to melt, then the mixed and pulverized components (9) to (13) were added and stirred to disperse them homogeneously. The homogeneously dissolved components (15) to (17), in which the component (14) was dispersed, were added, then the overall mixture was homogeneously emulsified, then filled in a container and cooled to obtain the solid water-in-oil type emulsion foundation.

Each of the solid water-in-oil type emulsion cosmetic composition obtained in the above Examples 4 to 6 was evaluated by a panel in the same way as in Example 1. As a result, each was evaluated as being "Excellent" in all items other than the items evaluated as "Good".

As explained above, the solid water-in-oil type emulsion cosmetic composition according to the present invention is an epochmaking solid water-in-oil type emulsion cosmetic composition not seen in the past which is capable of containing a relatively large amount of water, has a fresh, superior, skin feeling, and effectively corrects skin roughness, while exhibiting a long-lasting power.

## Claims

1. A solid water-in-oil type emulsion cosmetic composition comprising a hydrophobically treated powder, spherical silica, an oil, and water, a ratio by weight of the oil to the water (i.e., oil/water) being 0.5 to 10.

2. A solid water-in-oil type emulsion cosmetic composition as claimed in claim 1, wherein the amount of the spherical silica blended is 2.0 to 15.0 % by weight and the proportion of the spherical silica in the total spherical powder is 30.0 to 100 % by weight.

3. A solid water-in-oil type emulsion cosmetic composition as claimed in claim 1, wherein the total amount of the oil and water is 40.0 to 90.0 % by weight.

4. A solid water-in-oil type emulsion cosmetic composition as claimed in claim 1, wherein the ratio by weight of the oil to the water (i.e., oil/water) is 1 to 5.

5. A solid water-in-oil type emulsion cosmetic composition as claimed in claim 1, wherein the cosmetic composition is a make-up cosmetic composition.
